# EUROPÄISCHE PATENTSCHRIFT

(11) **EP 2 640 718 B1**
(45) Veröffentlichungstag und Bekanntmachung des Hinweises auf die Patenterteilung: **28.03.2018**
(21) Anmeldenummer: 11784992.7
(22) Anmeldetag: 15.11.2011
(51) Int. Cl.: C07D 403/14, A61K 31/506, A61P 9/00

(54) **SUBSTITUIERTES NATRIUM-1H-PYRAZOL-5-OLAT**
SUBSTITUTED SODIUM-1H-PYRAZOL-5-OLATE
SODIUM-1H-PYRAZOL-5-OLATE SUBSTITUE

(30) Priorität: 18.11.2010 DE 102010044131
(43) Veröffentlichungstag der Anmeldung: 25.09.2013
(73) Patentinhaber: Bayer Intellectual Property GmbH, 40789 Monheim (DE)
(72) Erfinder: MILITZER, Hans-Christian, 51519 Odenthal (DE); GRIES, Jörg, 42781 Haan (DE); KOEP, Stefan, 58256 Ennepetal (DE)
(74) Vertreter: BIP Patents
(86) Internationale Anmeldenummer: PCT/EP2011/070099
(87) Internationale Veröffentlichungsnummer: WO 2012/065967

(56) Entgegenhaltungen:
- WO-A1-2008/067871

## Beschreibung

Die vorliegende Anmeldung betrifft Natrium-1-[6-(morpholin-4-yl)pyrimidin-4-yl]-4-(1H-1,2,3-triazol-1-yl)-1H-pyrazol-5-olat, Verfahren zu dessen Herstellung, dessen Verwendung zur Behandlung und/oder Prophylaxe von Krankheiten sowie dessen Verwendung zur Herstellung von Arzneimitteln zur Behandlung und/oder Prophylaxe von Krankheiten, insbesondere von kardiovaskulären und hämatologischen Erkrankungen, von Nierenerkrankungen sowie zur Förderung der Wundheilung.

Die Verbindung der Formel (I), 1-[6-(Morpholin-4-yl)pyrimidin-4-yl]-4-(1H-1,2,3-triazol-1-yl)-1H-pyrazol-5-ol (Enol-Form; Formel (Ia)) bzw. 2-[6-(Morpholin-4-yl)pyrimidin-4-yl]-4-(1H-1,2,3-triazol-1-yl)-1,2-dihydro-3H-pyrazol-3-on (Keto-Form; Formel (Ib)), ist aus WO 2008/067871 bekannt.

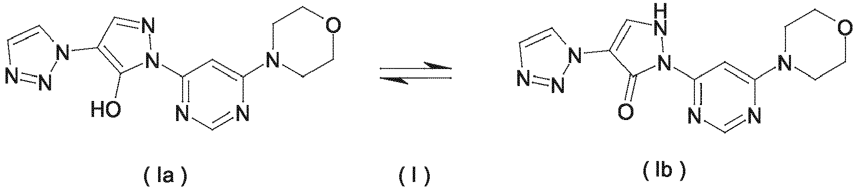

Die Verbindung der Formel (I) wirkt als Hemmstoff der HIF-Prolyl-4-Hydroxylasen und führt aufgrund dieses spezifischen Wirkmechanismus *in vivo* nach parenteraler oder oraler Verabreichung die Induktion von HIF-Zielgenen, wie z.B. Erythropoetin, und der hierdurch verursachten biologischen Prozesse, wie z.B. Erythropoese, herbei.

Die Verbindung der Formel (I) ist hygroskopisch und nimmt bei üblichen Umgebungsbedingungen (20-35°C, Normaldruck) bereits oberhalb einer relativen Luftfeuchtigkeit von 20 %rF bis zu etwa 6 Gew% Wasser auf. Die Aufnahme von 6 Gew% Wasser ist bei einer Luftfeuchte von 30 %rF nahezu erreicht. Eine Wasser enthaltende Verbindung der Formel (I) gibt bei abnehmender Luftfeuchte unterhalb von 30 %rF Teile des enthaltenen Wassers wieder ab. Diese Wasseraufnahme beziehungsweise Wasserabgabe erschweren den Umgang mit der Verbindung der Formel (I), z. B. Wägeprozesse, und die Herstellung der Verbindung der Formel (I) in einer gleichbleibenden, stabilen und definierten Form für den Einsatz in Arzneimitteln beziehungsweise die Herstellung von Arzneimitteln enthaltend die Verbindung der Formel (I). Insbesondere erhöht sich dadurch der technische Aufwand bei der Herstellung von Applikationsformen enthaltend die Verbindung der Formel (I), wie z.B. Tabletten, Granulaten oder Trinklösungen, da für eine gleichbleibende Konzentration der Verbindung der Formel (I) Maßnahmen zur Kontrolle und zur Regelung der Luftfeuchtigkeit erforderlich sind.

Es wurde nun überraschend gefunden, dass aus der Verbindung der Formel (I) ein Natriumsalz hergestellt werden kann, das gegenüber der Verbindung der Formel (I) entscheidende Vorteile aufweist.

Gegenstand der vorliegenden Erfindung ist die Verbindung Natrium-1-[6-(morpholin-4-yl)pyrimidin-4-yl]-4-(1H-1,2,3-triazol-1-yl)-1H-pyrazol-5-olat entsprechend der Verbindung der Formel (II)

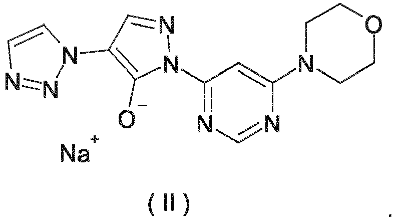

Bevorzugt im Rahmen der vorliegenden Erfindung liegt Natrium-1-[6-(morpholin-4-yl)pyrimidin-4-yl]-4-(1H-1,2,3-triazol-1-yl)-1H-pyrazol-5-olat (Verbindung der Formel (II)) in kristalliner Form vor.

Durch den erfindungsgemäßen Einsatz der Verbindung der Formel (II) wird sichergestellt, dass eine im Vergleich zur bekannten Verbindung der Formel (I) signifikant höhere Stabilität gegenüber der Aufnahme beziehungsweise Abgabe von Wasser bei schwankender Luftfeuchtigkeit erreicht wird. Natrium-1-[6-(morpholin-4-yl)pyrimidin-4-yl]-4-(1H-1,2,3-triazol-1-yl)-1H-pyrazol-5-olat (Verbindung der Formel (II)) enthält weniger als 0,5 Gew% Wasser, ist nicht hygroskopisch und ändert unter üblichen Umgebungsbedingungen (20-35°C, Normaldruck) auch bei erhöhter Luftfeuchte bis zu 90 %rF seinen Wassergehalt nur minimal, d. h. weniger als 0,5 Gew%. Die Verbindung der Formel (II) ist technisch deutlich leichter handhabbar, d. h. bei Wägeprozessen, und insbesondere wenn es darum geht, eine gleichbleibende Konzentration der Verbindung der Formel (II) in einer Applikationsform, wie z.B. einem Granulat, einer Trinklösung oder einer Tablette, zu gewährleisten. Darüber hinaus weist die Verbindung der Formel (II) gegenüber der Verbindung der Formel (I) eine erhöhte Löslichkeit in Wasser auf.

Weiterer Gegenstand der Erfindung ist ein Verfahren zur Herstellung der erfindungsgemäßen Verbindung der Formel (II), dadurch gekennzeichnet, dass die Verbindung der Formel (I) 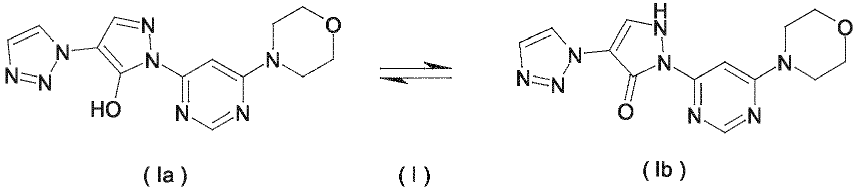 in einem Lösungsmittel mit Natriumhydroxid oder wässriger Natronlauge oder einem Natrium-Salz, gegebenenfalls unter Zusatz einer Base, umgesetzt wird.

Bevorzugt ist ein Verfahren zur Herstellung der erfmdungsgemäßen Verbindung der Formel (II), dadurch gekennzeichnet, dass die Verbindung der Formel (I) in einem Lösungsmittel mit wässriger Natronlauge, gegebenenfalls unter Zusatz einer Base, umgesetzt wird.

Besonders bevorzugt ist ein Verfahren zur Herstellung der erfmdungsgemäßen Verbindung der Formel (II), dadurch gekennzeichnet, dass die Verbindung der Formel (I) in einem Lösungsmittel mit wässriger Natronlauge, unter Zusatz von Triethylamin, umgesetzt wird.

Die Umsetzung mit Natriumhydroxid oder wässriger Natronlauge oder einem Natrium-Salz erfolgt im Allgemeinen in einem Lösungsmittel, bevorzugt in einem Temperaturbereich von 20°C bis 120°C, besonders bevorzugt in einem Temperaturbereich von 40°C bis 70°C, bei Normaldruck. Die Verbindung der Formel (II) wird aus der erhaltenen Suspension bei einer Temperatur zwischen -20°C und 80°C, bevorzugt bei einer Temperatur von 0°C bis 20°C, bei Normaldruck durch Filtration isoliert und anschließend getrocknet.

Findet die Umsetzung unter Zusatz einer Base statt, wird die Verbindung der Formel (I) im Allgemeinen zunächst unter Zugabe einer organischen Base in einem Lösungsmittel bei einer Temperatur von 20°C bis 120°C, bevorzugt bei einer Temperatur von 40°C bis 70°C, bei Normaldruck gelöst und anschließend die Verbindung der Formel (II) durch Zugabe von Natriumhydroxid oder wässriger Natronlauge oder einem Natrium-Salz bei einer Temperatur von 20°C bis 120°C, bevorzugt bei einer Temperatur von 40°C bis 70°C, bei Normaldruck gefällt. Die Verbindung der Formel (II) wird aus der erhaltenen Suspension bei einer Temperatur zwischen -20°C und 80°C, bevorzugt bei einer Temperatur von 0°C bis 20°C, bei Normaldruck durch Filtration isoliert und anschließend getrocknet.

Natriumhydroxid und wässrige Natronlauge und das Natrium-Salz werden in einem Molverhältnis von 0,8 bis 2 Moläquivalenten bezüglich der Verbindung der Formel (I) eingesetzt. Bevorzugt werden Natriumhydroxid und wässrige Natronlauge und das Natrium-Salz in einem Molverhältnis von 1,0 bis 1,4 Moläquivalenten bezüglich der Verbindung der Formel (I) eingesetzt.

Als Natrium-Salz eigenen sich beispielsweise Salze organischer Säuren wie Natriumcarboxylate, wie beispielsweise Natriumacetat oder Natriumcitrat, oder Salze anorganischer Säuren wie beispielsweise Natriumcarbonat, Natriumhydrogencarbonat, Natriumphosphat, Natriumhydrogenphosphat oder Natriumchlorid.

Als Lösungsmittel eignen sich niedere Alkohole wie beispielsweise Methanol, Ethanol, n-Propanol, iso-Propanol, n-Butanol, sec-Butanol, iso-Butanol, 1-Pentanol, oder Tetrahydrofuran, oder Acetonitril, oder Toluol, oder 1,4-Dioxan oder Gemische der genannten Lösungsmittel, oder Gemische der genannten Lösungsmittel mit Wasser. Bevorzugt sind Methanol, Ethanol, 2-Propanol, Tetrahydrofuran oder Gemische der genannten Lösungsmittel mit Wasser. Besonders bevorzugt sind Mischungen von Methanol mit Wasser oder Ethanol mit Wasser in einem Verhältnis zwischen 1 : 1 und 50 : 1 (v/v), ganz besonders bevorzugt sind Mischungen von Methanol mit Wasser in einem Verhältnis zwischen 7 : 3 und 30 : 1 (v/v).

Als organische Basen eignen sich tertiäre Amine wie beispielsweise Triethylamin oder Diisopropylethylamin. Bevorzugt ist Triethylamin. Die organische Base wird in einem Verhältnis von 0 bis 4 Moläquivalenten bezüglich der Verbindung der Formel (I) eingesetzt. Bevorzugt wird die organische Base in einem Verhältnis von 0,7 bis 1,5 Moläquivalenten bezüglich der Verbindung der Formel (I) eingesetzt.

Die Herstellung der erfindungsgemäßen Verbindung der Formel (II) kann durch das folgende Reaktionsschema veranschaulicht werden:

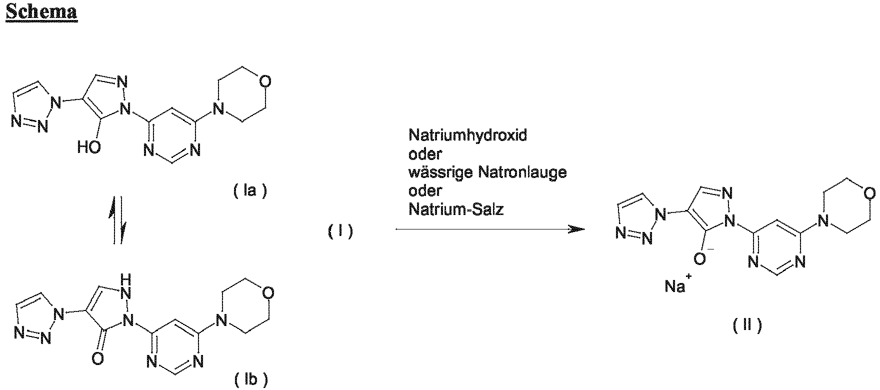

### Erklärungen zu den Abbildungen:

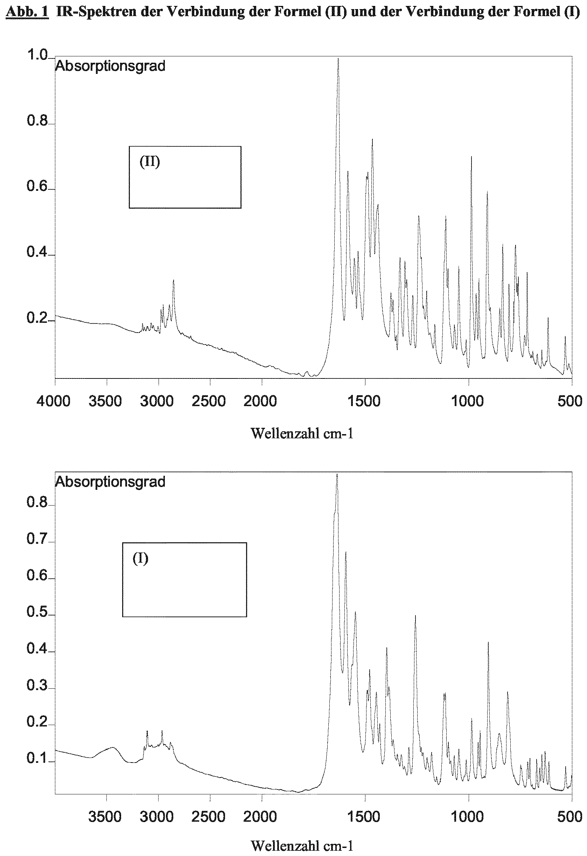

Abbildung 1: IR-Spektren der Verbindung der Formel (II) und der Verbindung der Formel (I)

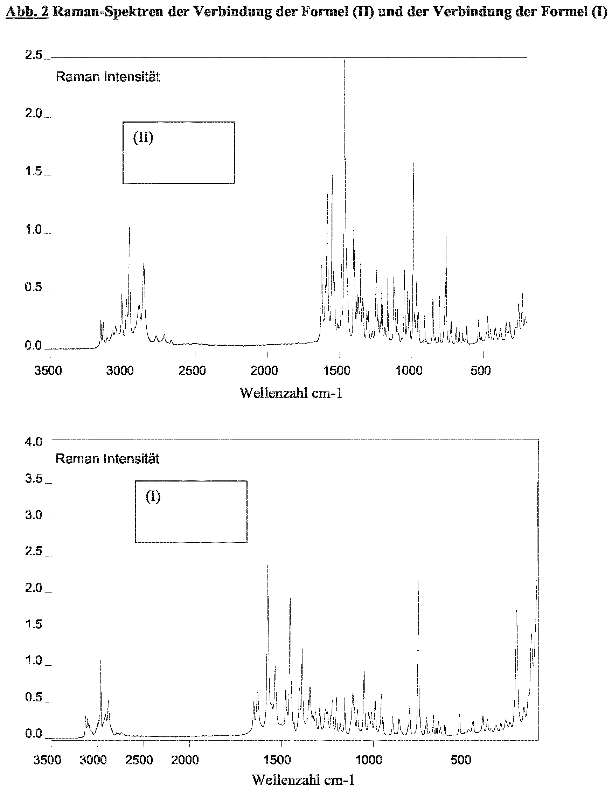

Abbildung 2: Raman-Spektren der Verbindung der Formel (II) und der Verbindung der Formel (I)

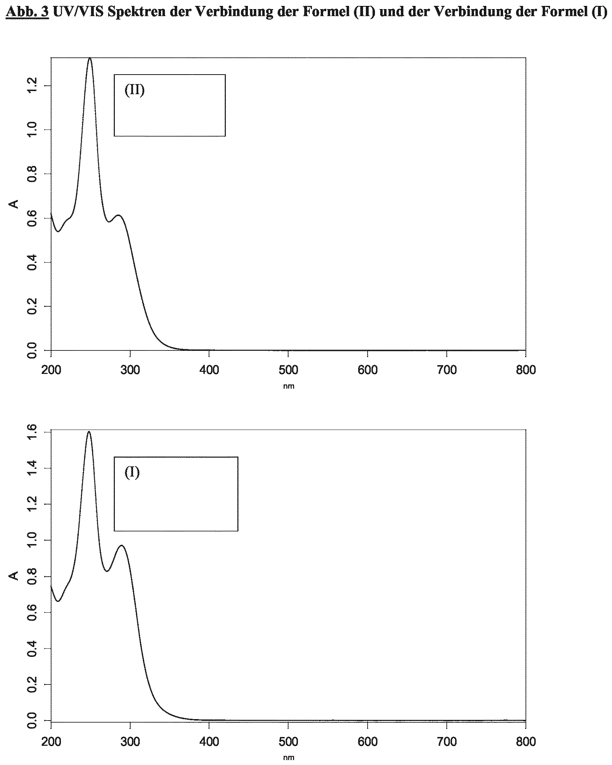

Abbildung 3: UV/VIS Spektren der Verbindung der Formel (II) und der Verbindung der Formel (I)

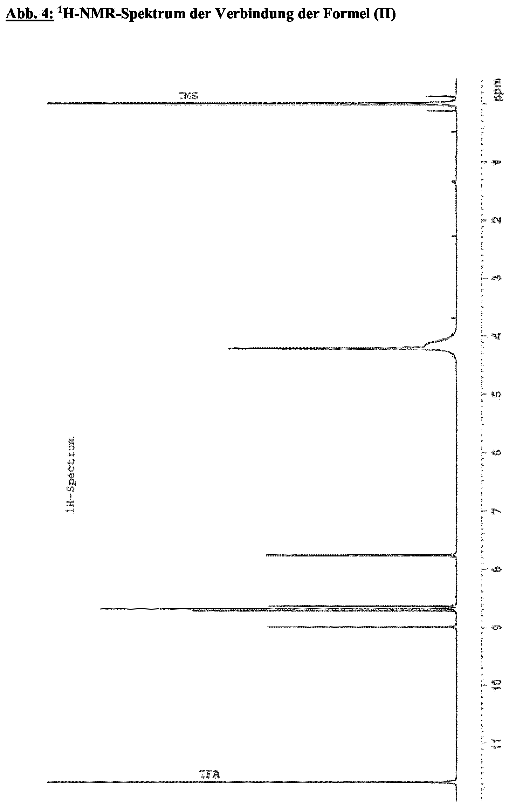

Abbildung 4: ¹H-NMR-Spektrum der Verbindung der Formel (II)

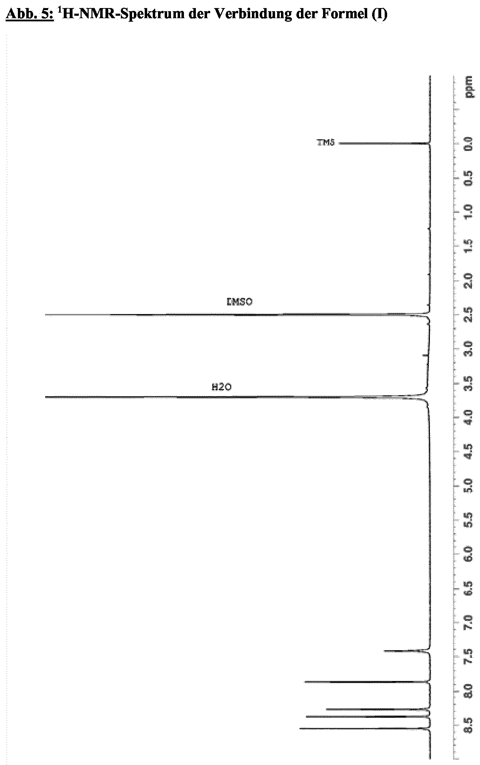

Abbildung 5: ¹H-NMR-Spektrum der Verbindung der Formel (I)

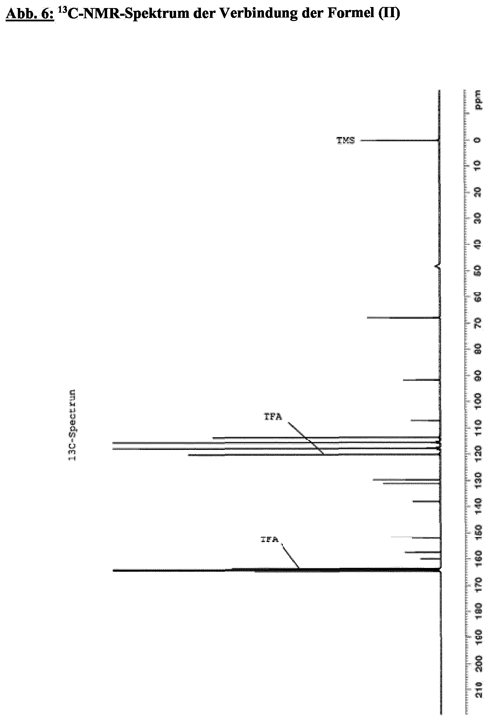

Abbildung 6: ¹³C-NMR-Spektrum der Verbindung der Formel (II)

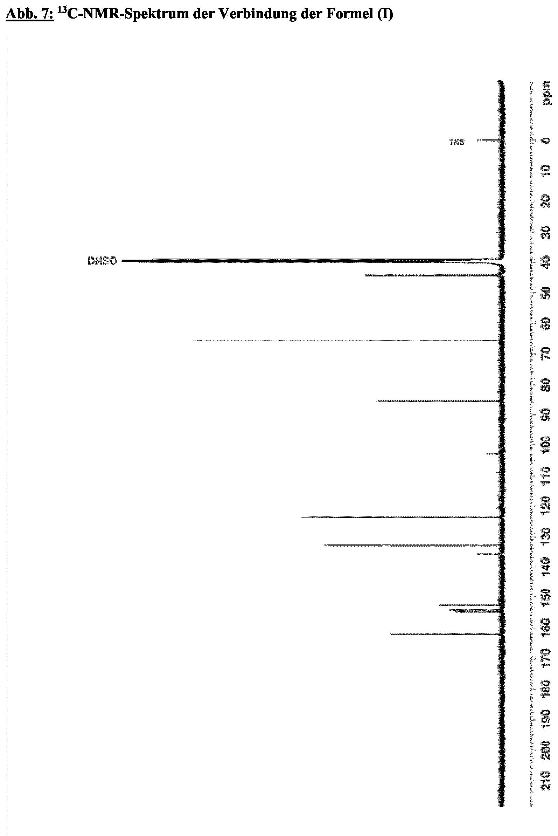

Abbildung 7: ¹³C-NMR-Spektrum der Verbindung der Formel (I)

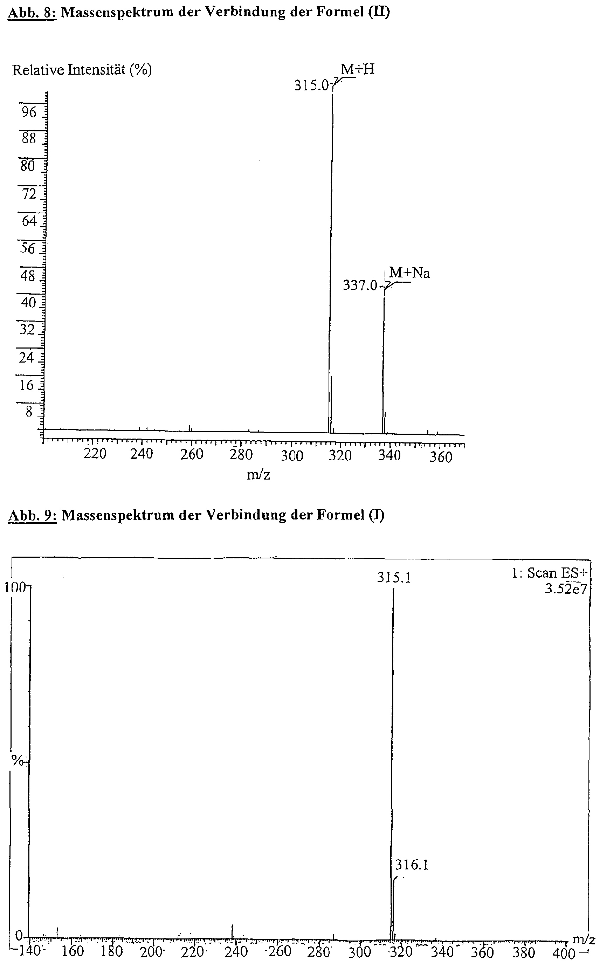

Abbildung 8: Massenspektrum der Verbindung der Formel (II)
Abbildung 9: Massenspektrum der Verbindung der Formel (I)

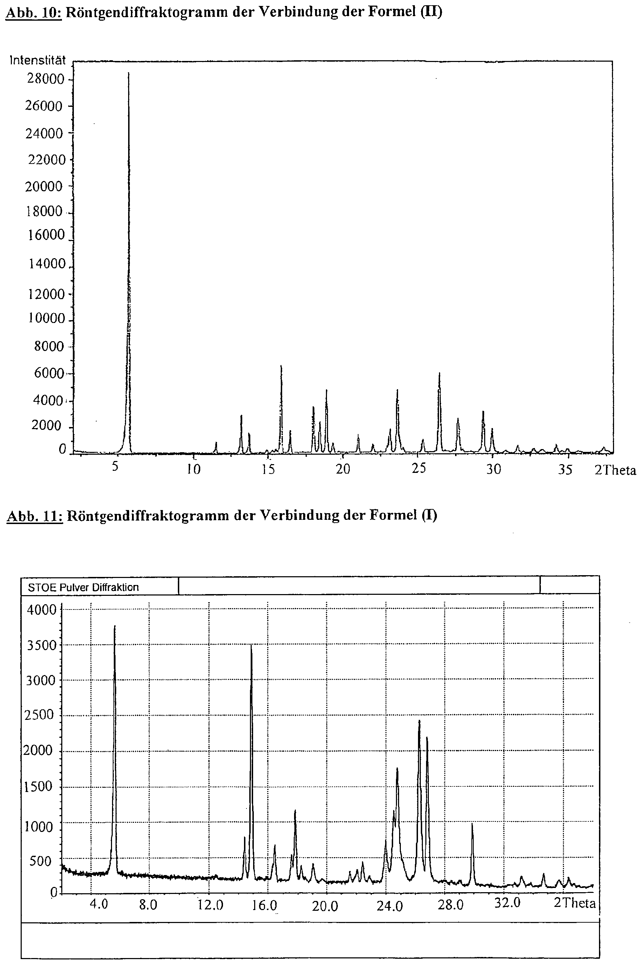

Abbildung 10: Röntgendiffraktogramm der Verbindung der Formel (II)
Abbildung 11: Röntgendiffraktogramm der Verbindung der Formel (I)

Die erfindungsgemäße Verbindung der Formel (II) zeigt ein nicht vorhersehbares, wertvolles pharmakologisches Wirkspektrum. Sie eignet sich daher zur Verwendung als Arzneimittel zur Behandlung und/oder Prophylaxe von Krankheiten bei Menschen und Tieren.

Die erfindungsgemäße Verbindung der Formel (II) zeichnet sich als spezifischer Hemmstoff der HIF-Prolyl-4-Hydroxylasen aus.

Die erfindungsgemäße Verbindung der Formel (II) kann aufgrund ihrer pharmakologischen Eigenschaften zur Behandlung und/oder Prophylaxe von kardiovaskulären Krankheiten, insbesondere von Herzinsuffizienz, koronarer Herzkrankheit, Angina pectoris, Myokardinfarkt, Schlaganfall, Arteriosklerose, essentieller, pulmonaler und maligner Hypertonie sowie peripherer arterieller Verschlusskrankheit eingesetzt werden.

Die erfindungsgemäße Verbindung der Formel (II) eignet sich weiterhin zur Behandlung und/oder Prophylaxe von Störungen der Blutbildung, wie z.B. idiopathischen Anämien, renaler Anämie und Anämien in Begleitung einer Tumor-Erkrankung (insbesondere einer Chemotherapie-induzierten Anämie), einer Infektion (insbesondere HIV-Infektion) oder einer anderen entzündlichen Erkrankung wie z.B. rheumatoider Arthritis. Die erfindungsgemäße Verbindung der Formel (II) ist darüber hinaus geeignet zur unterstützenden Behandlung von Anämien infolge Blutverlust, Eisenmangel-Anämie, Vitaminmangel-Anämie (z.B. infolge Vitamin B12-Mangel oder infolge Folsäure-Mangel), hypoplastischer und aplastischer Anämie, hämolytischer Anämie oder zur unterstützenden Behandlung von Anämien in Folge von Eisenverwertungsstörungen (sideroachrestische Anämie) oder Anämien infolge anderer endokriner Störungen (z.B. Hypothyreose).

Die erfindungsgemäße Verbindung der Formel (II) ist ferner geeignet zur Steigerung des Hämatokrits mit dem Ziel der Gewinnung von Blut zur Eigenblutspende vor Operationen.

Die erfindungsgemäße Verbindung der Formel (II) kann außerdem zur Behandlung und/oder Prophylaxe von operationsbedingten Ischämiezuständen und deren Folgeerscheinungen nach chirurgischen Eingriffen, insbesondere Eingriffen am Herzen unter Verwendung einer Herz-Lungenmaschine (z.B. Bypass-Operationen, Herzklappen-Implantationen), Eingriffen an den Halsschlagadern, Eingriffen an der Körperschlagader (Aorta) und Eingriffen mit instrumenteller Öffnung oder Durchdringung der Schädelkalotte verwendet werden. Die erfindungsgemäße Verbindung der Formel (II) eignet sich ferner zur allgemeinen Behandlung und/oder Prophylaxe bei chirurgischen Eingriffen mit dem Ziel einer Beschleunigung der Wundheilung und Verkürzung der Rekonvaleszenzzeit.

Die erfindungsgemäße Verbindung der Formel (II) ist darüber hinaus zur Behandlung und Prophylaxe von Folgeerscheinungen akuter und protrahierter Ischämiezustände des Gehirns geeignet (z.B. Schlaganfall, Geburtsasphyxie).

Die erfindungsgemäße Verbindung der Formel (II) kann ferner zur Behandlung und/oder Prophylaxe von Krebs und zur Behandlung und/oder Prophylaxe einer im Zuge der Behandlung von Krebs auftretenden Beeinträchtigung des Gesundheitszustandes insbesondere nach Therapie mit Zytostatika, Antibiotika und Bestrahlungen eingesetzt werden.

Die erfindungsgemäße Verbindung der Formel (II) eignet sich ferner zur Behandlung und/oder Prophylaxe von Erkrankungen des rheumatischen Formenkreises und anderen den Autoimmunerkrankungen zuzurechnenden Krankheitsformen und insbesondere zur Behandlung und/oder Prophylaxe einer im Zuge der medikamentösen Behandlung derartiger Erkrankungen auftretenden Beeinträchtigung des Gesundheitszustandes.

Weiterhin kann die erfindungsgemäße Verbindung der Formel (II) eingesetzt werden zur Behandlung und/oder Prophylaxe von Erkrankungen des Auges (z.B. Glaukom), des Gehirns (z.B. Morbus Parkinson, Morbus Alzheimer, Demenz, chronisches Schmerzempfinden), von chronischen Nierenerkrankungen, Niereninsuffizienz und akutem Nierenversagen sowie zur Förderung der Wundheilung.

Weiterhin eignet sich die erfindungsgemäße Verbindung der Formel (II) zur Behandlung und/oder Prophylaxe einer insbesondere im höheren Lebensalter gehäuft auftretenden allgemeinen körperlichen Schwäche bis hin zur Kachexie.

Ferner eignet sich die erfindungsgemäße Verbindung der Formel (II) zur Behandlung und/oder Prophylaxe sexueller Dysfunktion.

Außerdem ist die erfindungsgemäße Verbindung der Formel (II) zur Behandlung und/oder Prophylaxe von Diabetes mellitus und seinen Folgeerkrankungen, wie z.B. diabetischer Makro- und Mikroangiopathie, diabetischer Nephropathie und Neuropathie, geeignet.

Weiterhin eignet sich die erfindungsgemäße Verbindung der Formel (II) zur Behandlung und/oder Prophylaxe von fibrotischen Erkrankungen z.B. des Herzens, der Lunge und der Leber.

Insbesondere ist die erfindungsgemäße Verbindung der Formel (II) auch zur Prophylaxe und Behandlung der Frühgeborenenretinopathie (Retinopathia praematurorum) geeignet.

Weiterer Gegenstand der vorliegenden Erfindung ist die Verwendung der erfindungsgemäßen Verbindung der Formel (II) zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindung der Formel (II) zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge der erfindungsgemäßen Verbindung der Formel (II).

Die erfindungsgemäße Verbindung der Formel (II) kann allein oder bei Bedarf in Kombination mit anderen Wirkstoffen eingesetzt werden. Weiterer Gegenstand der vorliegenden Erfindung sind Arzneimittel, enthaltend die erfindungsgemäße Verbindung der Formel (II) und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt: ACE-Inhibitoren, Angiotensin II-Rezeptor-Antagonisten, Beta-Rezeptor-Blocker, Calcium-Antagonisten, PDE-Inhibitoren, Mineralocorticoid-Rezeptor-Antagonisten, Diuretika, Aspirin, Eisen-Supplements, Vitamin B12- und Folsäure-Supplements, Statine, Digitalis (Digoxin)-Derivate, Tumor-Chemotherapeutika sowie Antibiotika.

Bei einer bevorzugten Ausführungsfrom der Erfindung wird die erfindungsgemäße Verbindung der Formel (II) in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung wird die erfindungsgemäße Verbindung der Formel (II) in Kombination mit einem Angiotensin All-Antagonisten, wie beispielhaft und vorzugsweise Losartan, Candesartan, Valsartan, Telmisartan oder Embusartan, verabreicht.

Bei einer bevorzugten Ausführungsfrom der Erfindung wird die erfindungsgemäße Verbindung der Formel (II) in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung wird die erfindungsgemäße Verbindung der Formel (II) in Kombination mit einem Calcium-Antagonisten, wie beispielhaft und vorzugsweise Nifedipin, Amlodipin, Verapamil oder Diltiazem, verabreicht.

Bei einer bevorzugten Ausführungsfrom der Erfindung wird die erfindungsgemäße Verbindung der Formel (II) in Kombination mit einem Phosphodiesterase (PDE)-Inhibitor, wie beispielhaft und vorzugsweise Milrinone, Amrinone, Pimobendan, Cilostazol, Sildenafil, Vardenafil oder Tadalafil, verabreicht.

Bei einer bevorzugten Ausführungsform der Erfindung wird die erfindungsgemäße Verbindung der Formel (II) in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung wird die erfindungsgemäße Verbindung der Formel (II) in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsfrom der Erfindung wird die erfindungsgemäße Verbindung der Formel (II) in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsfrom der Erfindung wird die erfindungsgemäße Verbindung der Formel (II) in Kombination mit einem Tumor-Chemotherapeutikum verabreicht, beispielhaft und vorzugsweise aus der Gruppe der Platin-Komplexe, wie z.B. Cisplatin und Carboplatin, der Alkylantien, wie z.B. Cyclophosphamid und Chlorambucil, der Antimetabolite, wie z.B. 5-Fluoruracil und Methotrexat, der Topoisomerase-Hemmer, wie z.B. Etoposid und Camptothecin, der Antibiotika, wie z.B. Doxorubicin und Daunorubicin, oder der Kinase-Inhibitoren, wie z.B. Sorafenib und Sunitinib.

Bei einer bevorzugten Ausführungsfrom der Erfindung wird die erfindungsgemäße Verbindung der Formel (II) in Kombination mit einem Antibiotikum verabreicht, beispielhaft und vorzugsweise aus der Gruppe der Penicilline, Cephalosporine oder Chinolone, wie z.B. Ciprofloxacin und Moxifloxacin.

Weiterer Gegenstand der vorliegenden Erfindung sind Arzneimittel, die die erfindungsgemäße Verbindung der Formel (II), üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäße Verbindung der Formel (II) kann systemisch und/oder lokal wirken. Zu diesem Zweck kann sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege kann die erfindungsgemäße Verbindung der Formel (II) in geeigneten Applikationsformen verabreicht werden.

Für die orale Applikation eignen sich nach dem Stand der Technik funktionierende, die erfindungsgemäße Verbindung der Formel (II) schnell und/oder modifiziert abgebende Applikationsformen, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäße Verbindung der Formel (II) kann in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung etwa 0,01 bis 100 mg/kg, vorzugsweise etwa 0,01 bis 20 mg/kg und ganz besonders bevorzugt 0,1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- ACN: Acetonitril
- bS: breites Singulett (in NMR Spektren)
- D: Dublett (in NMR Spektren)
- DSC: Dynamische Differenzkalorimetrie (Differential Scanning Calorimetry)
- Gew%: Gewichtsprozent
- M: Multiplett (in NMR Spektren)
- n.d.: nicht detektiert
- NMR: Kernmagnetische Resonanz (nuclear magnetic resonance)
- rF: relative Feuchte
- S: Singulett (in NMR Spektren)
- sec: Sekunden
- T: Triplett (in NMR Spektren)
- v/v: Volumen/Volumen
- δ: Deformationsschwingungen
- v: Streckschwingungen

### Ausgangsverbindungen:

### Beispiel 1A

### 2-(1H-1,2,3-Triazol-1-yl)acrylsäuremethylester

450 g 2-(1*H*-1,2,3-Triazol-1-yl)essigsäureethylester wurden in 3,5 l Methanol gelöst, mit 30 g Triethylamin versetzt und 16 h bei 22°C gerührt. Anschließend wurden im Vakuum alle Lösungsmittel abdestilliert. Man erhielt 410 g der Titelverbindung als Öl.

### Beispiel 2A

### 3-(Dimethylamino)-2-(1H-1,2,3-triazol-1-yl)acrylsäuremethylester

400 g 2-(1H-1,2,3-Triazol-1-yl)acrylsäuremethylester wurden mit 522 g Dimethylformamiddimethylacetal versetzt und zum Sieden erhitzt. Entstehende Leichtsieder wurden abdestilliert. Nach 4 h wurde auf 55°C abgekühlt und 1050 ml Methyl-tert-butylether / 2-Propanol (3 : 1 v/v) wurden zudosiert. Die erhaltene Suspension wurde auf 22°C abgekühlt und filtriert. Der Filterkuchen wurde mehrfach mit Methyl-tert-butylether gewaschen und im Vakuum bei 40°C getrocknet. Man erhielt 493 g der Titelverbindung als Feststoff.

### Beispiel 3A

### 1-[6-(Morpholin-4-yl)pyrimidin-4-yl]-4-(1H-1,2,3-triazol-1-yl)-1H-pyrazol-5-ol (Enol-Form; Formel (Ia)) bzw. 2-[6-(Morpholin-4-yl)pyrimidin-4-yl]-4-(1H-1,2,3-triazol-1-yl)-1,2-dihydro-3H-pyrazol-3-on (Keto-Form; Formel (Ib))

20 g 4-(6-Hydrazinopyrimidin-4-yl)morpholin und 24,6 g (2E/Z)-3-(Dimethylamino)-2-(1H-1,2,3-triazol-1-yl)acrylsäuremethylester in 210 ml Ethylacetat wurden mit 5,84 g Trifluoressigsäure versetzt und 24 h unter Rückfluss gerührt. Die erhaltene Suspension wurde auf 0°C abgekühlt und filtriert. Der Filterkuchen wurde mit Ethylacetat gewaschen, scharf abgesaugt und anschließend in 160 ml Wasser suspendiert. Die Suspension wurde durch Zugabe von 4,5 ml Essigsäure auf etwa pH 5 eingestellt, weitere 15 Minuten gerührt und filtriert. Der Filterkuchen wurde zweimal mit 50 ml Wasser gewaschen und anschließend im Vakuum bei 40°C getrocknet. Ausbeute: 26,0 g (79,4 % d. Th.) der Titelverbindung.

Die Herstellung der Verbindungen 4-(6-Hydrazinopyrimidin-4-yl)morpholin (Beispiel Nr. 16A), 2-(1*H*-1,2,3-Triazol-1-yl)essigsäureethylester (Beispiel Nr. 39A) und 3-(Dimethylamino)-2-(1*H-*1,2,3-triazol-1-yl)acrylsäureethylester (Beispiel Nr. 3A) wurde bereits in WO 2008/067871 beschrieben.

Die Herstellung der Verbindung 2-(6-Morpholin-4-ylpyrimidin-4-yl)-4-(1*H*-1,2,3-triazol-1-yl)-1,2-dihydro-3*H-*pyrazol-3-on aus 4-(6-Hydrazinopyrimidin-4-yl)morpholin und 3-(Dimethylamino)-2-(1*H*-1,2,3-triazol-1-yl)acrylsäureethylester wurde ebenfalls in WO 2008/067871 beschrieben (Beispiel Nr. 71).

### Ausführungsbeispiele:

### Beispiel 1

### Natrium-1-[6-(morpholin-4-yl)pyrimidin-4-yl]-4-(1H-1,2,3-triazol-1-yl)-1H-pyrazol-5-olat (Verbindung der Formel (II))

### Beispiel 1.1

10 g der Verbindung aus Beispiel 3A wurden in 50 ml Methanol/Wasser (9 : 1 v/v) suspendiert. Zu der Suspension wurden 3,4 g 45%ige Natronlauge unter Rühren gegeben und weitere 50 ml Methanol/Wasser (9 : 1 v/v) hinzugefügt. Die Suspension wurde auf 50°C erwärmt und 2 h bei 50°C gerührt. Anschließend wurde auf 0°C abgekühlt, 1 h bei 0°C nachgerührt und filtriert. Der erhaltene Filterkuchen wurde mit Methanol/Wasser (9 : 1 v/v) gewaschen und getrocknet. Ausbeute: 10,1 g der Verbindung der Formel (II); 6,8 Gew% Na

### Beispiel 1.2

5 g der Verbindung aus Beispiel 3A wurden in 60 ml Ethanol/Wasser (1 : 1 v/v) suspendiert und bei 22°C mit 1,41 g 45%iger Natronlauge versetzt. Die Suspension wurde 3 Tage bei 50°C und 2 h bei 20°C gerührt. Der Feststoff wurde abfiltriert, mit 10 ml Wasser gewaschen und getrocknet. Ausbeute: 4 g der Verbindung der Formel (II).

### Beispiel 1.3

30,25 g der Verbindung aus Beispiel 3A wurden in 150 ml Methanol/Wasser (9 : 1 v/v) bei 22°C suspendiert. 13,3 ml Triethylamin wurden zugegeben und die Mischung wurde auf 60°C erwärmt. Nach 15 min. wurde die erhaltene fast klare Lösung filtriert, der Filter mit 10 ml Methanol/Wasser (9 : 1 v/v) nachgewaschen und das gesammelte Filtrat bei 60°C langsam mit 10,3 g 45%iger Natronlauge versetzt. Die erhaltene Suspension wird mit wenigen Kristallen der Verbindung der Formel (II) versetzt, 1 h bei 60°C nachgerührt, anschließend langsam auf 0°C abgekühlt und filtriert. Der Filterkuchen wird mit 15 ml Methanol/Wasser (9 : 1 v/v) gewaschen und im Vakuum bei 40°C getrocknet. Ausbeute: 25,1 g der Verbindung der Formel (II).

### Beispiel 1.4

25 g der Verbindung aus Beispiel 3A wurden in 150 ml Methanol/Wasser (1 : 1 v/v) suspendiert und mit 11 ml Triethylamin versetzt. Die erhaltene Lösung wurde auf 60°C erwärmt und mit 8,5 g 45%iger Natronlauge versetzt. Die erhaltene Suspension wurde langsam auf 22°C abgekühlt, 2 h bei 22°C und anschließend 1 h bei 0-5°C gerührt. Nach Filtration wurde der Filterkuchen mit 15 ml Methanol/Wasser (1 : 1 v/v) gewaschen und im Vakuum bei 40°C getrocknet. Ausbeute: 26 g der Verbindung der Formel (II).

### Dynamische Differenzkalorimetrie (DSC):

Die Thermogramme wurden unter Verwendung eines Differential Scanning Calorimeters DSC 7 bzw. Pyris-1 und Thermogravimetric Analyser TGA 7 der Fa. Perkin-Elmer erhalten.

*Differential Scanning Calorimeter DSC 7 bzw. Pyris-1;* Hersteller: Perkin-Elmer; Heizrate: 2 und 20 K/min: Spülgas: Stickstoff; Tiegel: nicht gasdichte Aluminium-Tiegel; Probenvorbereitung: keine.

*Thermogravimetric Analyzer TGA 7;* Hersteller: Perkin-Elmer; Heizrate: 10 Kmin⁻¹; Spülgas: Stickstoff, 20-30 ml/min; Tiegel: offener Platin-Tiegel; Probenvorbereitung: keine.

Natrium-1-[6-(morpholin-4-yl)pyrimidin-4-yl]-4-(1H-1,2,3-triazol-1-yl)-1H-pyrazol-5-olat (Verbindung der Formel (II)) zersetzt sich oberhalb von 300°C ohne zu schmelzen.

### Wasserdampfadsorption und Wasserdampfdesorption:

Die Feuchtesorptionsisotherme wurde mit einem Dynamic Vapour Sorption Analyzer IGA Sorp der Fa. Hiden Analytical aufgenommen. Die Messtemperatur betrug 25°C. Es gab keine Probenvorbereitung.

**Tabelle 1: Wasserdampfadsorption und Wasserdampfdesorption von Natrium-1-[6-(morpholin-4-yl)pyrimidin-4-yl]-4-(1H-1,2,3-triazol-1-yl)-1H-pyrazol-5-olat (Verbindung der Formel (II))**

| | Verbindung der Formel (II) | | Verbindung der Formel (I) | |
|---|---|---|---|---|
| Relative | Adsorption | Desorption | Adsorption | Desorption |
| 0 %rF | 0 % | 0 % | 0,1 % | 0,1 % (5 %rF) |
| 10 %rF | 0 % | 0 % | 0,2 % | 0,1 % |
| 20 %rF | 0,1 % | 0 % | 0,3 % | 0,2 % |
| 30 %rF | 0,1 % | 0,1 % | 4,8 % | 4,7 % |
| 40 %rF | 0,2 % | 0,2 % | 5,1 % | 5,2 % |
| 50 %rF | 0,2 % | 0,3 % | 5,3 % | 5,4 % |
| 60 %rF | 0,2 % | 0,3 % | 5,4 % | 5,6 % |
| 70 %rF | 0,4 % | 0,4 % | 5,6 % | 5,6 % |
| 80 %rF | 0,4 % | 0,5 % | 5,7 % | 5,7 % |
| 90 %rF | 0,8 % | 0,8 % | 5,9 % | 5,9 % |

### Löslichkeitsdaten:

Methode: Gesättigte Lösungen der Prüfsubstanz wurden durch 16stündiges Rühren einer Suspension in Wasser bei 25°C hergestellt. Die erhaltenen Suspensionen wurden anschließend filtriert und der Gehalt im Filtrat wurde mittels HPLC-Messung bestimmt.

**Tabelle 2: Löslichkeiten in Wasser**

| | Verbindung der Formel (II) | Verbindung der Formel (I) |
|---|---|---|
| Löslichkeit in Wasser bei 25°C [mg/100ml] | 2800 | 14,3 |

### IR and Raman Spektroskopie:

Für die Messung von IR- und Raman-Spektren der Verbindung der Formel (II) wurden Bruker FT/IR-Spektrometer IFS 66v und Bruker FT/Raman-Spektrometer MultiRAM mit folgenden Parametern verwendet:

| | **IR** | **Raman** |
|---|---|---|
| Spektrale Auflösung | 2 cm⁻¹ | 2 cm⁻¹ |
| Anzahl der Einzelmessungen (Scans) | 32 | 64 |
| Wellenzahlbereich | 4000 - 500 cm⁻¹ | 3500 - 200 cm⁻¹ |
| Probenvorbereitung | KBr Pressling | keine |

**Tabelle 3: Zuordnung charakteristischer Schwingungsbanden in den IR- bzw. Raman-Spektren der Verbindung der Formel (II)**

| Strukturelement | IR-Bandenlage (cm⁻¹) | Raman-Bandenlage (cm⁻¹) |
|---|---|---|
| v =C-H | 3153 - 3006 | 3153 - 3010 |
| v C-H | 2976 - 2855 | 2978 - 2856 |
| v C=C, v C=N | 1630 - 1439 | 1623 - 1401 |
| v C-N | 1241 | 1244 |
| v C-O | 1112 | 1118 |
| δ =C-H _{in phase} | 987 | 988 |

Für die Messung von IR- und Raman-Spektren der Verbindung der Formel (I) wurden Bruker FT/IR-Spektrometer Vertex 80v und Bruker FT/Raman-Spektrometer MultiRAM mit folgenden Parametern verwendet:

| | **IR** | **Raman** |
|---|---|---|
| Spektrale Auflösung | 2 cm⁻¹ | 2 cm⁻¹ |
| Anzahl der Einzelmessungen (Scans) | 32 | 54 |
| Wellenzahlbereich | 4000 - 500 cm⁻¹ | 3500 - 100 cm⁻¹ |
| Probenvorbereitung | KBr Pressling | keine |

**Tabelle 4: Zuordnung charakteristischer Schwingungsbanden in den IR- bzw. Raman-Spektren der Verbindung der Formel (I)**

| Strukturelement | IR-Bandenlage (cm⁻¹) | Raman-Bandenlage (cm⁻¹) |
|---|---|---|
| v O-H | 3441 | - |
| v =C-H | 3135 - 3108 | 3134 - 3006 |
| v C-H | 2965 - 2884 | 2967 - 2884 |
| v C=C, v C=N, δ C-H | 1636 - 1345 | 1650 - 1345 |
| v C-O_{Ether} | 1257 | 1259 |

### UV/VIS-Spektroskopie:

UV/VIS-Spektren wurden auf einem Perkin Elmer Spektrometer (Lambda 40P) mit den folgenden Bedingungen bzw. Parametern gemessen:
Küvette: Durchgangslänge 1cm; Quartzglas
Wellenzahlbereich: 200 - 800 nm
Öffnungsspalt: 1 nm
Probenvorbereitung: Ca. 1 mg/100ml Acetonitril/Wasser 1:1
Banden: 285; 249 nm Verbindung der Formel (II) und 289,3; 248,2 nm Verbindung der Formel (I)

**Tabelle 5: Berechnung der spezifischen Absorption und des molaren Absorptionskoeffizienten**

| Verbindung der Formel | Lösungsmittel | Wellenlänge (nm) | Spezifische Absorption A^{1%}_{1cm} (liter/g * cm) | Molarer Absorptionskoeffizent ε (liter/mol * cm) |
|---|---|---|---|---|
| (I) | Acetonitril/ Wasser 1:1 | 249 | 1111 | 34928 |
| (II) | Acetonitril/ Wasser 1:1 | 284 | 501.2 | 16855 |

### NMR-Spektroskopie:

NMR-Spektren wurden auf einem Bruker NMR-Spektrometer (Advance) mit folgenden Bedingungen bzw. Parametern aufgenommen:

| Verbindung der Formel (II) | ¹H-NMR-Spektrum | ¹³C-NMR-Spektrum |
|---|---|---|
| Arbeitsfrequenz | 500.13 MHz | 125.76 MHz |
| Lösungsmittel | Trifluoressigsäure | Trifluoressigsäure |
| Konzentration | 6.84 mg/ml | 42.7 mg/ml |
| Interner Standard | Tetramethylsilan (TMS) | Tetramethylsilan (TMS) |
| Probenröhrchen-Durchmesser | 5 mm | 5 mm |
| Temperatur | 25°C | 25°C |
| Technik | Fouriertransformationstechnik | Fouriertransformationstechnik |
| Spektrale Breite | 20.65 ppm | 245.41 ppm |
| Digitale Auflösung | 0.079 Hz/Pt | 0.4710 Hz/Pt |
| Pulsdauer | 2.83 µsec, 30° Anregungswinkel | 9.1 µsec, 90° Anregungswinkel |
| Aufhahmedauer | 6.399 sec | 1.06 sec |
| Relaxationszeit | 0.5 sec | 4 sec |
| No. of free induction decays | 32 | 128 |

| Verbindung der Formel (I) | ¹H-NMR-Spektrum | ¹³C-NMR-Spektrum |
|---|---|---|
| Arbeitsfrequenz | 500.13 MHz | 125.76 MHz |
| Lösungsmittel | Dimelhylsufoxid-d₆ (DMSO) | Dimethylsulfoxid-d₆ (DMSO) |
| Konzentration | 6.3 mg/ml | 35.8 mg/ml |
| Interner Standard | Tetramethylsilan (TMS) | Tetramethylsilan (TMS) |
| Probenröhrchen-Durchmesser | 5 mm | 5 mm |
| Temperatur | 25°C | 27°C |
| Technik | Fouriertransformationstechnik | Fouriertransformationstechnik |
| Spektrale Breite | 20.65 ppm | 240.89 ppm |
| Digitale Auflösung | 0.079 Hz/Pt | 0.9248 Hz/Pt |
| Pulsdauer | 3.1 µsec, 30° Anregungswinkel | 7.0 µsec, 90° Anregungswinkel |
| Aufhahmedauer | 6.344 sec | 1.08 sec |
| Relaxationszeit | 0.5 sec | 4 sec |
| No. of free induction decays | 32 | 1024 |

Strukturformeln der Verbindung der Formel (II) und der Verbindung der Formel (I) mit Zuordnung der jeweiligen NMR-Signale

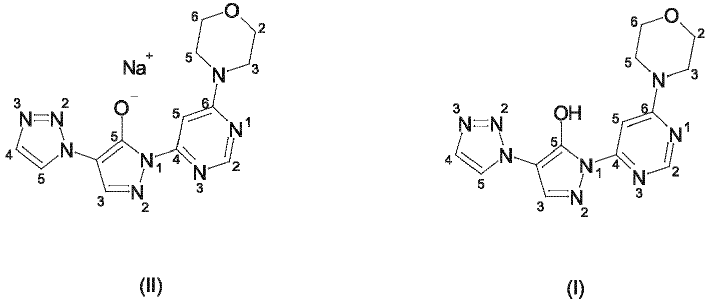

**Tabelle 6: ¹H-NMR-Spektrum der Verbindung der Formel (II) - Chemische Verschiebung, Signalmultiplizität, relative Protonenanzahl (Nummerierung der H-Atome bezieht sich auf die Strukturformel zur Zuordnung der jeweiligen NMR-Signale)**

| H-Atome | Chem. Verschiebung δ (ppm) | Multiplizität und Kopplungskonstanten | Anzahl der Protonen /Molekül |
|---|---|---|---|
| Natrium-1-[6-(morpholin-4-yl) | | | |
| H-2; H-3; H-5; H-6 | 4.00-4.25 | M | 8 |
| | | | |
| pyrimidin-4-yl]- | | | |
| H-2 | 8.72 | S | 1 |
| H-5 | 7.77 | S | 1 |
| | | | |
| 4-(1H-1,2,3-triazol-1-yl)- | | | |
| H-4 | 8.64 | D J= 1.4 Hz | 1 |
| H-5 | 8.98 | D J= 1.4 Hz | 1 |
| | | | |
| 1H-pyrazol-5-olat | | | |
| H-3 | 8.68 | S | 1 |

**Tabelle 7: ¹H-NMR-Spektrum der Verbindung der Formel (I) - Chemische Verschiebung, Signalmultiplizität, relative Protonenanzahl (Nummerierung der H-Atome bezieht sich auf die Strukturformel zur Zuordnung der jeweiligen NMR-Signale)**

| H-Atome | Chem. Verschiebung δ (ppm) | Multiplizität und Kopplungskonstanten | Anzahl der Protonen /Molekül |
|---|---|---|---|
| 1-[6-(morpholin-4-yl) | | | |
| H-2; H-3; H-5; H-6 | 3.71 | S | 8 |
| | | | |
| pyrimidin-4-yl]- | | | |
| H-2 | 8.55 | S | 1 |
| H-5 | 7.42 | S | 1 |
| | | | |
| 4-(1H-1,2,3-triazol-1-yl)- | | | |
| H-4 | 7.86 | D (0.6Hz) | 1 |
| H-5 | 8.38 | D (0.6Hz) | 1 |
| | | | |
| 1H-pyrazol-5-ol | | | |
| H-3 | 8.27 | S | 1 |
| 5-OH | n.d (c) | n.d. | 1 |

**Tabelle 8: ¹³C-NMR-Spektrum der Verbindung der Formel (II) - Chemische Verschiebung, Signalmultiplizität, relative C-Kernzahl in der Verbindung der Formel (II) (Nummerierung der C-Atome bezieht sich auf die Strukturformel zur Zuordnung der jeweiligen NMR-Signale)**

| C-Atome | Chem. Verschiebung δ (ppm) | Multiplizität und Kopplungskonstanten | Anzahl der C-Kerne /Molekül |
|---|---|---|---|
| Natrium-1-[6-(morpholin-4-yl) | | | |
| C-2; C-6 | 67.80 | T | 2 |
| C-3; C-5 | 48.21 | T | 2 |
| | | | |
| pyrimidin-4-yl]- | | | |
| C-2 | 151.65 | D | 1 |
| C-4 | 152.01 | S | 1 |
| C-5 | 91.48 | D | 1 |
| C-6 | 159.90 | S | 1 |
| | | | |
| 4-(1H-1,2,3-triazol-1-yl)- | | | |
| C-4 | 130.93 | D | 1 |
| C-5 | 129.57 | D | 1 |
| | | | |
| 1H-pyrazol-5-olat | | | |
| C-3 | 138.03 | D | 1 |
| C-4 | 106.99 | S | 1 |
| C-5 | 157.43 | S | 1 |

**Tabelle 9: ¹³C-NMR-Spektrum der Verbindung der Formel (I) - Chemische Verschiebung, Signalmultiplizität, relative C-Kernzahl in der Verbindung der Formel (I) (Nummerierung der C-Atome bezieht sich auf die Strukturformel zur Zuordnung der jeweiligen NMR-Signale)**

| C-Atome | Chem. Verschiebung δ (ppm) | Multiplizität und Kopplungskonstanten | Anzahl der C-Kerne /Molekül |
|---|---|---|---|
| 1-[6-(morpholin-4-yl | | | |
| C-2; C-6 | 65.56 | T | 2 |
| C-3, C-5 | 44.29 | T | 2 |
| | | | |
| pyrimidin-4-yl]- | | | |
| C-2 | 154.08 | D | 1 |
| C-4 | 152.43 | S | 1 |
| C-5 | 85.62 | D | 1 |
| C-6 | 161.99 | S | 1 |
| | | | |
| 4-(1H-1,2,3-triazol-1-yl)- | | | |
| C-4 | 132.94 | D | 1 |
| C-5 | 123.68 | D | 1 |
| | | | |
| 1H-pyrazol-5-ol | | | |
| C-3 | 135.84 | D | 1 |
| C-4 | 102.82 | S | 1 |
| C-5 | 154.70 | S | 1 |

### Massenspektroskopie:

Das Massenspektrum wurde auf einem Waters Massenspektrometer (ZQ) mit den nachfolgend aufgeführten Bedingungen bzw. Parametern aufgenommen:

| | |
|---|---|
| Ionisationsmethode | ESI (Electronic Spray Ionization) |
| Lösungsmittel | Acetonitril/Wasser |

**Tabelle 10: Interpretation des Massenspektrums der Verbindung der Formel (II)**

| | | Massenzahl (MZ) |
|---|---|---|
| Ionenbildung M+H | C13H14N8O2 + H | 315 |
| Ionenbildung M+Na | C13H14N8O2 + Na | 337 |

**Tabelle 11: Interpretation des Massenspektrums der Verbindung der Formel (I)**

| | | Massenzahl (MZ) |
|---|---|---|
| Ionenbildung M+H | C13H15N8O2 | 315 |

### Elementaranalyse:

**Tabelle 12: Ergebnisse der Elementanalyse der Verbindung der Formel (II)**

| Element | gemessen (%) | berechnet (%) | Differenz (%) |
|---|---|---|---|
| C | 46.1 | 46.4 | 0.3 |
| H | 4.0 | 3.9 | 0.1 |
| N | 33.1 | 33.3 | 0.2 |

**Tabelle 13: Ergebnisse der Elementanalyse der Verbindung der Formel (I)**

| Element | gemessen (%) | berechnet (%) | Differenz (%) |
|---|---|---|---|
| C | 49.5 | 49.7 | 0.2 |
| H | 4.4 | 4.5 | 0.1 |
| N | 35.5 | 35.7 | 0.2 |
| O | 12.6 | 10.2 | 2.4 |

### Röntgendiffraktometrie:

Transmissionsdiffraktometer PANalytical X'Pert PRO mit PIXcel-Zähler (Multichannel):

| | |
|---|---|
| Strahlung: | Kupfer, K alpha |
| Primär-Monochromator: | fokussierender Röntgenspiegel |
| Wellenlänge (K1): | 1,5406 Å |
| Wellenlänge (K2): | 1,5444 Å |
| Generatorparameter: | 40 kV, 40 mA |
| Messbereich: | 2-38° |
| Raumbedingungen: | 25°C, 40 - 60 %rF |

bzw.

STOE Pulver Diffraktions-System:

| | |
|---|---|
| Diffraktometer : | Transmission |
| Monochromator : | Curved Germanium (111) |
| Generator : | 45 kV, 35 mA |
| Wellenlänge : | 1.540598 Cu |
| Detektor : | Linear PSD |
| Scan Mode : | Transmission / Moving PSD / Fixed omega |
| Scan Type : | 2Theta:Omega |
| Raumbedingungen: | 25°C, 40 - 60 %rF |

**Tabelle 14: Pulverröntgendiffraktometrie der Verbindung der Formel (II)**

| Verbindung der Formel (II) | |
|---|---|
| Reflexe [2 Theta] | |
| 5,7 | 23,6 |
| 11,5 | 25,3 |
| 13,2 | 26,4 |
| 13,7 | 26,9 |
| 15,8 | 27,7 |
| 16,4 | 27,7 |
| 18,4 | 29,4 |
| 18,9 | 29,9 |
| 19,3 | 30,0 |
| 21,0 | 30,7 |
| 22,0 | 31,5 |
| 23,1 | 31,6 |

**Tabelle 15: Pulverröntgendiffraktometrie der Verbindung der Formel (I)**

| Verbindung der Formel (I) | |
|---|---|
| Reflexe [2 Theta] | |
| 5,6 | 24,6 |
| 14,4 | 24,8 |
| 14,9 | 25,1 |
| 16,3 | 26,3 |
| 16,5 | 26,8 |
| 17,6 | 28 |
| 17,8 | 28,5 |
| 18,2 | 29 |
| 18,5 | 29,9 |
| 19 | 32,4 |
| 19,7 | 32,7 |
| 21,6 | 33,2 |
| 22 | 33,8 |
| 22,4 | 34,7 |
| 22,9 | 35,7 |
| 24 | 36,4 |
| 24,5 | 36,7 |

### B. Bewertung der pharmakologischen Wirksamkeit

Die pharmakologischen Eigenschaften der erfindungsgemäßen Verbindungen können in folgenden Assays gezeigt werden:

### Abkürzungen:

- DMEM: Dulbecco's Modified Eagle Medium
- FCS: Fetal Calf Serum
- TMB: 3,3',5,5'-Tetramethylbenzidin
- Tris: Tris(hydroxymethyl)-aminomethan

### 1. In vitro-Tests zur Bestimmung der Aktivität und Selektivität von HIF-Prolyl-4-Hydroxylase-Inhibitoren

### 1.a) Hemmung der Aktivität von HIF-Prolylhydroxylase:

Hydroxylierter HIF bindet spezifisch an den von Hippel-Lindau Protein-Elongin B-Elongin C-Komplex (VBC-Komplex). Diese Interaktion tritt nur auf, wenn HIF an einem konservierten Prolylrest hydroxyliert ist. Sie ist die Grundlage für die biochemische Bestimmung der HIF-Prolylhydroxylase-Aktivität. Der Test wird wie beschrieben durchgeführt [Oehme F., Jonghaus W., Narouz-Ott L., Huetter J., Flamme I., Anal. Biochem. 330 (1), 74-80 (2004)]:
Eine klare, mit NeutrAvidin HBC beschichtete 96-Loch-Mikrotiterplatte (Fa. Pierce) wird für 30 Minuten mit Blocker-Casein inkubiert. Anschließend wird die Platte dreimal mit je 200 µl Waschpuffer (50 mM Tris, pH 7.5, 100 mM NaCl, 10% (v/v) Blocker-Casein, 0.05% (v/v) Tween 20) pro Loch gewaschen. Das Peptid Biotin-DLDLEMLAPYIPMDDDFQL (Fa. Eurogentec, 4102 Seraing, Belgien) wird in einer Konzentration von 400 nM in 100 µl Waschpuffer zugegeben. Dieses Peptid dient als Substrat für die Prolylhydroxylierung und wird an die Mikrotiterplatte gebunden. Nach 60 Minuten Inkubation wird die Platte dreimal mit Waschpuffer gewaschen, 30 Minuten mit 1 mM Biotin in Blocker-Casein inkubiert und dann erneut dreimal mit Waschpuffer gewaschen.

Zur Durchführung der Prolylhydroxylase-Reaktion wird das an die Platte gebundene Peptidsubstrat 1 bis 60 Minuten mit einem Prolylhydroxylase-haltigen Zelllysat inkubiert. Die Reaktion findet in 100 µl Reaktionspuffer (20 mM Tris, pH 7.5, 5 mM KCl, 1.5 mM MgCl₂, 1 µM-1 mM 2-Oxoglutarat, 10 µM FeSO₄, 2 mM Ascorbat) bei Raumtemperatur statt. Die Reaktionsmischung enthält außerdem den zu testenden Hemmstoff der Prolylhydroxylase in verschiedenen Konzentrationen. Die Testsubstanz wird bevorzugt, aber nicht ausschließlich bei Konzentrationen zwischen 1 nM und 100 µM eingesetzt. Durch dreimaliges Waschen der Platte mit Waschpuffer wird die Reaktion gestoppt.

Zur quantitativen Bestimmung der Prolylhydroxylierung wird ein Fusionsprotein, das sowohl Thioredoxin aus E. coli als auch den VBC-Komplex enthält, in 80 µl Bindungspuffer (50 mM Tris, pH 7.5, 120 mM NaCl) zugegeben. Nach 15 Minuten werden 10 µl einer Lösung von polyklonalem Anti-Thioredoxin-Antikörper aus Kaninchen in Bindungspuffer zugefügt. Nach weiteren 30 Minuten setzt man 10 µl einer Lösung von mit Meerrettich-Peroxidase gekoppeltem Anti-Kaninchen-Immunglobulin in Bindungspuffer hinzu. Nach 30 Minuten Inkubation bei Raumtemperatur wird dreimal mit Waschpuffer gewaschen, um ungebundenen VBC-Komplex und Antikörper zu entfernen. Um die Menge an gebundenem VBC-Komplex zu bestimmen, wird 15 Minuten mit TMB inkubiert. Die Farbreaktion wird durch Zugabe von 100 µl 1 M Schwefelsäure beendet. Durch Messung der optischen Dichte bei 450 nm wird die Menge an gebundenem VBC-Komplex bestimmt. Sie ist proportional zur Menge an hydroxyliertem Prolin im Peptidsubstrat.

Zur Detektion der Prolylhydroxylierung kann alternativ ein mit Europium (Fa. Perkin Elmer) gekoppelter VBC-Komplex verwendet werden. In diesem Fall wird die Menge an gebundenem VBC-Komplex durch zeitaufgelöste Fluoreszenz bestimmt. Außerdem ist die Verwendung von mit [³⁵S]-Methionin markiertem VBC-Komplex möglich. Hierfür kann der radioaktiv markierte VBC-Komplex durch *in vitro*-Transkription-Translation in Retikulozytenlysat hergestellt werden.

Die erfindungsgemäße Verbindung der Formel (II) hemmt die Aktivität der HIF-Prolylhydroxylase in diesem Test mit einem IC₅₀-Wert von 0,47 µM (Mittelwert für EGLN2/PHD1) bzw. 0,14 µM (Mittelwert für EGLN1/PHD2).

### 1.b) Zellulärer, funktioneller in vitro-Test:

Die Quantifizierung der Wirksamkeit der erfindungsgemäßen Verbindungen erfolgt mit Hilfe einer rekombinanten Zelllinie. Die Zelle leitet sich ursprünglich von einer humanen Lungencarcinom-Zelllinie ab (A549, ATCC: American Type Culture Collection, Manassas, VA 20108, USA). Die Testzelllinie wird stabil mit einem Vektor transfiziert, der das Reportergen der *Photinus pyralis-*Luciferase (im folgenden Luciferase genannt) unter der Kontrolle eines artifiziellen Minimalpromotors enthält. Der Minimalpromotor besteht aus zwei Hypoxie-responsiblen Elementen stromaufwärts einer TATA-Box [Oehme F., Ellinghaus P., Kolkhof P., Smith T.J., Ramakrishnan S., Hütter J., Schramm M., Flamme I., Biochem. Biophys. Res. Commun. 296 (2), 343-9 (2002)]. Unter Einwirkung von Hypoxie (z.B. Kultivierung in Gegenwart von 1% Sauerstoff für 24 Stunden) oder unter Einwirkung unselektiver Dioxygenase-Inhibitoren (z.B. Desferroxamin in einer Konzentration von 100 µM, Cobaltchlorid in einer Konzentration von 100 µM oder *N*-Oxalylglycindiethylester in einer Konzentration von 1 mM) produziert die Testzelllinie Luciferase, die mit Hilfe geeigneter Biolumineszenz-Reagenzien (z.B. Steady-Glo® Luciferase Assay System, Promega Corporation, Madison, WI 53711, USA) und eines geeigneten Luminometers detektiert und quantifiziert werden kann.

Testablauf: Die Zellen werden am Tag vor dem Test in einer exakt bemessenen Menge Kulturmedium (DMEM, 10% FCS, 2 mM Glutamin) in 384- oder 1536-Loch-Mikrotiterplatten ausplattiert und in einem Zellinkubator (96% Luftfeuchtigkeit, 5% v/v CO₂, 37°C) gehalten. Am Testtag werden dem Kulturmedium die Testsubstanzen in abgestuften Konzentrationen zugesetzt. In als Negativkontrolle dienenden Ansätzen wird den Zellen keine Testsubstanz zugesetzt. Als Positivkontrolle zur Bestimmung der Empfindlichkeit der Zelle für Inhibitoren wird z.B. Desferroxamin in einer Endkonzentration von 100 µM zugesetzt. Sechs bis 24 Stunden nach Übertragung der Testsubstanzen in die Löcher der Mikrotiterplatten wird das resultierende Lichtsignal im Luminometer gemessen. Anhand der Meßwerte wird eine Dosiswirkungsbeziehung aufgestellt, die als Grundlage für die Ermittlung der halbmaximalen Wirkkonzentration (als EC₅₀-Wert bezeichnet) dient.

Die erfindungsgemäße Verbindung der Formel (II) weist in dem hier beschriebenen Test einen EC₅₀-Wert von 7 µM auf.

### 1.c) Zellulärer, funktioneller in vitro-Test zur Veränderung der Genexpression:

Um die Veränderung der Expression spezifischer mRNAs in menschlichen Zelllinien nach Behandlung mit Testsubstanzen zu untersuchen, werden folgende Zelllinien auf 6- oder 24-Lochplatten kultiviert: humane Hepatomzellen (HUH, JCRB Cell Bank, Japan), humane embryonale Nierenfibroblasten (HEK/293, ATCC, Manassas, VA 20108, USA), humane Cervixcarcinomzellen (HeLa, ATCC, Manassas, VA 20108, USA), humane Nabelschnurvenenendothelzellen (HUVEC, Cambrex, East Rutherford, New Jersey 07073, USA). 24 Stunden nach Zugabe der Testsubstanzen werden die Zellen mit Phosphat-gepufferter Saline gewaschen und aus ihnen die Gesamt-RNA unter Verwendung einer geeigneten Methode gewonnen (z.B. Trizol®-Reagenz, Invitrogen GmbH, 76131 Karlsruhe, Deutschland).

Für ein typisches Analyseexperiment wird je 1 µg der so gewonnenen Gesamt-RNA mit DNase I verdaut und unter Verwendung einer geeigneten Reversen-Transkriptase-Reaktion (ImProm-II Reverse Transcription System, Promega Corporation, Madison, WI 53711, USA) in eine komplementäre DNA (cDNA) übersetzt. 2.5% des so gewonnenen cDNA-Ansatzes werden jeweils für die Polymerase-Kettenreaktion verwendet. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht. Die hierbei benutzten Primer-Sonden-Kombinationen werden mittels der Primer Express 1.5 Software (Fa. Applied Biosystems, Inc.) generiert. Im einzelnen werden die mRNAs von Erythropoetin, Carboanhydrase IX, Lactatdehydrogenase A und vaskulärem Endothelzellwachstumsfaktor untersucht.

### 2. In vivo-Tests zum Nachweis der Wirkung im Kardiovaskularsystem

### 2.a) In vivo-Test zur Veränderung der Genexpression:

Mäusen oder Ratten werden die in geeigneten Lösungsmitteln gelösten Prüfverbindungen entweder oral durch Schlundsonden-Applikation, intraperitoneal oder intravenös verabfolgt. Typische Dosierungen sind 0.1, 0.5, 1, 5, 10, 20, 50, 100 und 300 mg Substanz pro kg Körpergewicht und Verabfolgung. Kontrolltiere erhalten nur Lösungsmittel. 4, 8 oder 24 Stunden nach Gabe der Prüfsubstanz werden die Tiere mit einer Überdosis Isofluran und anschließendem Genickbruch getötet und die zu untersuchenden Organe entnommen. Teile der Organe werden in flüssigem Stickstoff schockgefroren. Aus den Organteilen wird wie unter B.1.a) beschrieben Gesamt-RNA gewonnen und diese in eine cDNA übersetzt. Das Expressionsniveau der mRNA der zu untersuchenden Gene wird mittels der *real time quantitative polymerase chain reaction* [TaqMan-PCR; Heid C.A., Stevens J., Livak K.J., Williams P.M., Genome Res. 6 (10), 986-94 (1996)] unter Verwendung eines ABI Prism 7700 Sequenz-Detektionsinstruments (Fa. Applied Biosystems, Inc.) untersucht.

Die Substanz gemäß der vorliegenden Erfindung führt im Vergleich mit der Placebo-Kontrolle nach oraler oder parenteraler Verabreichung zu einem signifikanten, dosisabhängigen Anstieg der mRNA des Erythropoetins in der Niere.

### 2.b) Bestimmung des Erythropoetin-Spiegels im Serum:

Mäusen oder Ratten wird die Prüfsubstanz in einem geeigneten Lösungsmittel entweder intraperitoneal oder oral einmal oder zweimal täglich verabreicht. Typische Dosierungen sind 0.1, 0.5, 1, 5, 10, 20, 50, 100 und 300 mg Substanz pro kg Körpergewicht und Verabfolgung. Placebo-Kontrolltiere erhalten nur Lösungsmittel. Vor der Applikation und vier Stunden nach der letzten Substanzgabe wird den Tieren in Kurznarkose aus dem retroorbitalen Venenplexus oder der Schwanzvene Blut entnommen. Das Blut wird durch Zusatz von Lithium-Heparin ungerinnbar gemacht. Durch Zentrifugieren wird das Blutplasma gewonnen. In dem Blutplasma wird mit Hilfe eines Erythropoetin-ELISA (Quantikine® mouse Epo Immunoassay, R&D Systems, Inc., Minneapolis, USA) entsprechend der Anleitung des Herstellers der Gehalt an Erythropoetin bestimmt. Die Meßwerte werden anhand einer für Maus-Erythropoetin erhobenen Referenzmessung in pg/ml umgerechnet.

Die Substanz gemäß der vorliegenden Erfindung führt nach oraler und parenteraler Verabreichung zu einem signifikanten, dosisabhängigen Anstieg des Plasma-Erythropoetins gegenüber dem Ausgangswert und der Placebo-Kontrolle.

### 2.c) Bestimmung der zellulären Zusammensetzung des peripheren Blutes:

Mäusen oder Ratten wird die Prüfsubstanz in einem geeigneten Lösungsmittel entweder intraperitoneal oder oral einmal oder zweimal täglich über mehrere Tage verabreicht. Typische Dosierungen sind z.B. 0.1, 0.5, 1, 5, 10, 20, 50, 100 und 300 mg Substanz pro kg Körpergewicht und Verabfolgung. Kontrolltiere erhalten nur Lösungsmittel. Am Versuchsende wird den Tieren in Kurznarkose aus dem Venenplexus des Augenwinkels oder der Schwanzvene Blut entnommen und durch Zusatz von Natriumcitrat ungerinnbar gemacht. In einem geeigneten elektronischen Messgerät werden in den Blutproben die Konzentrationen von Erythrozyten, Leukozyten und Thrombozyten bestimmt. Die Konzentration der Retikulozyten wird anhand von Blutausstrichen, die mit einer dafür geeigneten Farblösung (Fa. KABE Labortechnik, Nümbrecht) gefärbt werden, durch mikroskopische Durchmusterung von je 1000 Erythrozyten bestimmt. Für die Bestimmung des Hämatokrits wird Blut aus dem retroorbitalen Venenplexus mittels einer Hämatokritkapillare entnommen und der Hämatokritwert nach Zentrifugieren der Kapillare in einer dafür geeigneten Zentrifuge manuell abgelesen.

Die Substanz gemäß der vorliegenden Erfindung führt nach oraler und parenteraler Verabreichung zu einem signifikanten, dosisabhängigen Anstieg des Hämatokrits, der Erythrozytenzahl und der Retikulozyten gegenüber dem Ausgangswert und der Placebo-Kontrolle.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Natrium-1-[6-(morpholin-4-yl)pyrimidin-4-yl]-4-(1H-1,2,3-triazol-1-yl)-1H-pyrazol-5-olat entsprechend der Verbindung der Formel (II)

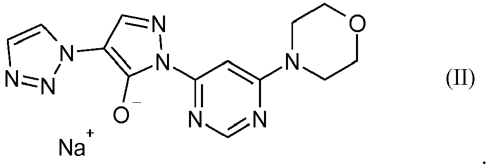

2. Verbindung der Formel (II) nach Anspruch 1, **dadurch gekennzeichnet, dass** die Verbindung der Formel (II) in kristalliner Form vorliegt.

3. Verfahren zur Herstellung der Verbindung der Formel (II) nach Anspruch 1, **dadurch gekennzeichnet, dass** die Verbindung der Formel (I) 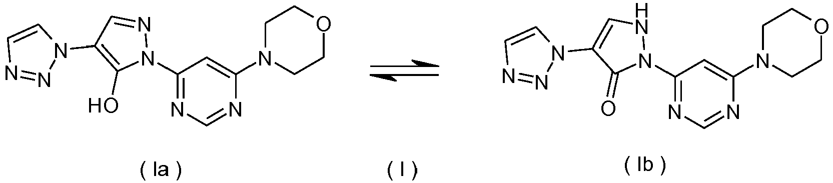 in einem Lösungsmittel mit Natriumhydroxid oder wässriger Natronlauge oder einem Natrium-Salz, gegebenenfalls unter Zusatz einer Base, umgesetzt wird.

4. Verfahren zur Herstellung der Verbindung der Formel (II) nach Anspruch 3, **dadurch gekennzeichnet, dass** die Verbindung der Formel (I) in einem Lösungsmittel mit wässriger Natronlauge, gegebenenfalls unter Zusatz einer Base, umgesetzt wird.

5. Verfahren zur Herstellung der Verbindung der Formel (II) nach Anspruch 3, **dadurch gekennzeichnet, dass** die Verbindung der Formel (I) in einem Lösungsmittel mit wässriger Natronlauge, unter Zusatz von Triethylamin, umgesetzt wird.

6. Verbindung nach einem der Ansprüche 1 oder 2 zur Behandlung und/oder Prophylaxe von Krankheiten.

7. Verwendung der Verbindung nach einem der Ansprüche 1 oder 2 zur Herstellung eines Arzneimittels zur Behandlung und/oder Prophylaxe von Krankheiten.

8. Verwendung der Verbindung nach einem der Ansprüche 1 oder 2 zur Herstellung eines Arzneimittels zur Behandlung und/oder Prophylaxe von Herz-Kreislauf-Erkrankungen, Herzinsuffizienz, Anämie, chronischen Nierenerkrankungen und Niereninsuffizienz.

9. Arzneimittel enthaltend die Verbindung nach einem der Ansprüche 1 oder 2 in Kombination mit einem inerten, nichttoxischen, pharmazeutisch geeigneten Hilfsstoff.

10. Arzneimittel enthaltend die Verbindung nach einem der Ansprüche 1 oder 2 in Kombination mit einem weiteren Wirkstoff.

11. Arzneimittel nach Anspruch 9 oder 10 zur Behandlung und/oder Prophylaxe von Herz-Kreislauf-Erkrankungen, Herzinsuffizienz, Anämie, chronischen Nierenerkrankungen und Niereninsuffizienz.

## Claims

1. Sodium 1-[6-(morpholin-4-yl)pyrimidin-4-yl]-4-(1H-1,2,3-triazol-1-yl)-1H-pyrazol-5-olate corresponding to the compound of the formula (II)

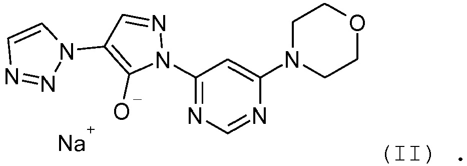

2. Compound of the formula (II) according to Claim 1, **characterized in that** the compound of the formula (II) is present in crystalline form.

3. Process for preparing the compound of the formula (II) according to Claim 1, **characterized in that** the compound of the formula (I) 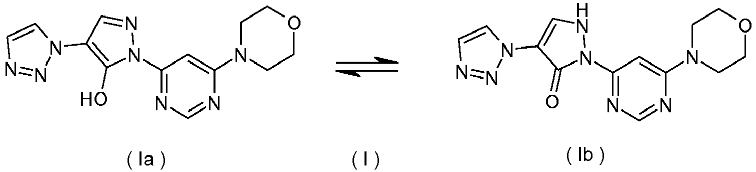 is reacted in a solvent with sodium hydroxide or aqueous sodium hydroxide solution or a sodium salt, if appropriate with addition of a base.

4. Process for preparing the compound of the formula (II) according to Claim 3, **characterized in that** the compound of the formula (I) is reacted in a solvent with aqueous sodium hydroxide solution, if appropriate with addition of a base.

5. Process for preparing the compound of the formula (II) according to Claim 3, **characterized in that** the compound of the formula (I) is reacted in a solvent with aqueous sodium hydroxide solution with addition of triethylamine.

6. Compound according to Claim 1 or 2 for the treatment and/or prophylaxis of diseases.

7. Use of the compound according to Claim 1 or 2 for the preparation of a medicament for treatment and/or prophylaxis of diseases.

8. Use of the compound according to Claim 1 or 2 for the preparation of a medicament for treatment and/or prophylaxis of cardiovascular diseases, cardiac insufficiency, anaemia, chronic kidney diseases and renal insufficiency.

9. Medicament comprising the compound according to Claim 1 or 2 in combination with an inert, nontoxic, pharmaceutically suitable auxiliary.

10. Medicament comprising the compound according to Claim 1 or 2 in combination with a further active compound.

11. Medicament according to Claim 9 or 10 for the treatment and/or prophylaxis of cardiovascular diseases, cardiac insufficiency, anaemia, chronic kidney diseases and renal insufficiency.

## Revendications

1. 1-[6-(Morpholin-4-yl)pyrimidin-4-yl]-4-(1H-1,2,3-triazol-1-yl)-1H-pyrazol-5-olate de sodium correspondant au composé de formule (II)

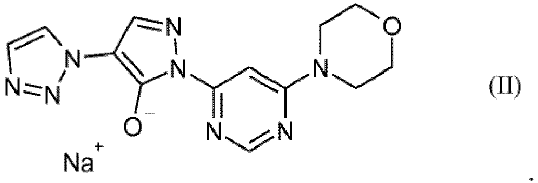

2. Composé de formule (II) selon la revendication 1, **caractérisé en ce que** le composé de formule (II) se présente sous forme cristalline.

3. Procédé de fabrication du composé de formule (II) selon la revendication 1, **caractérisé en ce que** le composé de formule (I) 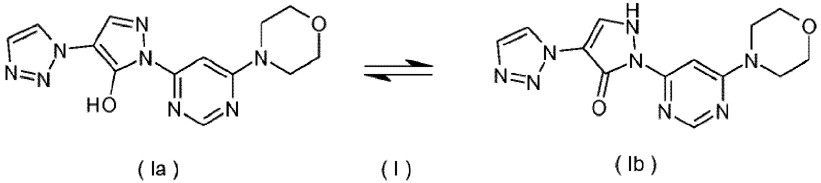 est mis en réaction dans un solvant avec de l'hydroxyde de sodium ou de la soude caustique aqueuse ou un sel de sodium, éventuellement avec ajout d'une base.

4. Procédé de fabrication du composé de formule (II) selon la revendication 3, **caractérisé en ce que** le composé de formule (I) est mis en réaction dans un solvant avec de la soude caustique aqueuse, éventuellement avec ajout d'une base.

5. Procédé de fabrication du composé de formule (II) selon la revendication 3, **caractérisé en ce que** le composé de formule (I) est mis en réaction dans un solvant avec de la soude caustique aqueuse, avec ajout de triéthylamine.

6. Composé selon l'une quelconque des revendications 1 ou 2, pour le traitement et/ou la prophylaxie de maladies.

7. Utilisation du composé selon l'une quelconque des revendications 1 ou 2 pour la fabrication d'un médicament pour le traitement et/ou la prophylaxie de maladies.

8. Utilisation du composé selon l'une quelconque des revendications 1 ou 2 pour la fabrication d'un médicament pour le traitement et/ou la prophylaxie de maladies cardio-vasculaires, de l'insuffisance cardiaque, de l'anémie, de maladies rénales chroniques et de l'insuffisance rénale.

9. Médicament contenant le composé selon l'une quelconque des revendications 1 ou 2 en combinaison avec un adjuvant inerte, non toxique, pharmaceutiquement approprié.

10. Médicament contenant le composé selon l'une quelconque des revendications 1 ou 2 en combinaison avec un autre agent actif.

11. Médicament selon la revendication 9 ou 10 pour le traitement et/ou la prophylaxie de maladies cardio-vasculaires, de l'insuffisance cardiaque, de l'anémie, de maladies rénales chroniques et de l'insuffisance rénale.
